(19) Europäisches Patentamt
European Patent Office
Office européen des brevets

(11) Publication number: **0 058 069**
**B1**

(12) # EUROPEAN PATENT SPECIFICATION

(45) Date of publication of patent specification: **27.08.86**

(21) Application number: **82300602.8**

(22) Date of filing: **08.02.82**

(51) Int. Cl.⁴: **C 07 D 213/65** // C07D213/89, C07D405/04, C07D491/04 ,(C07D491/04, 319:00, 221:00)

(54) **Process and intermediates for preparing pirbuterol and analogs.**

(30) Priority: **09.02.81 US 232923**

(43) Date of publication of application: **18.08.82 Bulletin 82/33**

(45) Publication of the grant of the patent: **27.08.86 Bulletin 86/35**

(84) Designated Contracting States: **AT BE CH DE FR GB IT LI LU NL SE**

(56) References cited:
**US-A-3 763 173**
**US-A-3 948 919**

**Can. J. Chem. 56, pp. 2673-2676, (1978)**

**Annalen der Chemie 613, pp. 144-153, (1958)**

**J. Chem. Soc., 1958, pp. 1263-1266**

(73) Proprietor: **PFIZER INC.**
**235 East 42nd Street**
**New York, N.Y. 10017 (US)**

(72) Inventor: **Cue, Berkeley Wendell, Jr.**
**266 Stoddards Wharf Drive**
**Gales Ferry Connecticut (US)**
Inventor: **Massett, Stephen Sargent**
**78 Brandegee Avenue**
**Groton Connecticut (US)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 058 069 B1

**Description**

This invention relates to processes for the preparation of pirbuterol and analogs, i.e. compounds of the formula

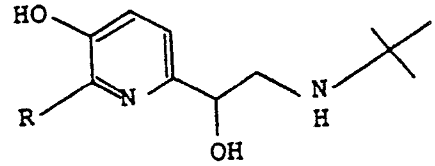

(I)

wherein R is hydrogen, methyl or hydroxymethyl, and certain intermediates therefor. Pirbuterol has the formula (I), wherein R is hydroxymethyl.

Pirbuterol and its bronchodilator activity were originally described by Barth in a series of U.S. Patents (3,700,681; 3,763,173; 3,772,314; 3,786,160). At that time, pirbuterol was synthesized as follows:

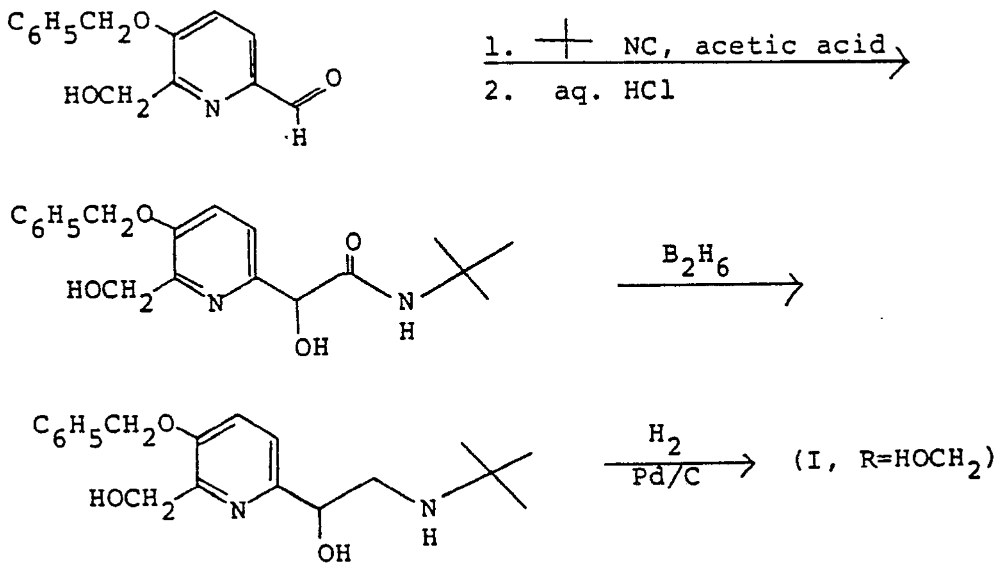


The pirbuterol analogs of the formula I, wherein R is hydrogen, methyl, or methanesulfonylmethyl, and their bronchodilator activity, were disclosed by Jen and Kaiser in U.S. Patent 3,952,101. These compounds were prepared from the corresponding aldehydes by a reaction sequence fully analogous with that of the earlier pirbuterol process.

Subsequently, an alternative, preferred process for the synthesis of pirbuterol was described by Nakanishi (U.S. Patents 3,948,919; 4,031,108), exemplified as follows:

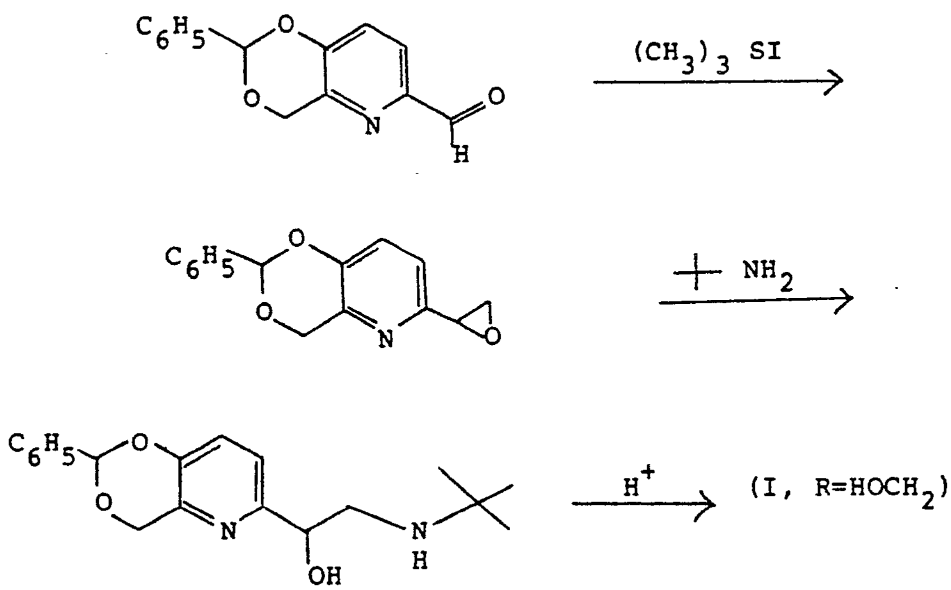

Improvements and alternatives to this process have also been described, *viz.*, isolation of the pirbuterol precursor as a maleate salt (Carroll *et al.*, U.S. Patent 4,011,231) and hydrogenolysis rather than hydrolysis as the final stage (Argentina Patent 214005, Luxembourg Patent 79564).

The latter process still suffers from the disadvantage of generating noxious sulfide by-products in the preparation of epoxide. Even under the best of conditions, traces of sulfur can carry through and increase the catalyst level when protecting groups are removed by hydrogenolysis, the preferred route when the free base form of pirbuterol is desired. Furthermore, the epoxide is relatively unreactive towards the *tert*-butylamine reagent, even under pressure at elevated temperatures requiring large excess of the reagent and a relatively long time to achieve complete reaction.

A further disadvantage of the improved process is the polar nature of intermediate amino alcohol, making this intermediate difficult to isolate in pure form.

Recently pirbuterol has been discovered to also have utility for the treatment of congestive heart failure (Taylor, U.S. Patent 4,175,128).

In a chemical transformation related to one of the process steps of the present invention, Benderly *et al.*, [Can. J. Chem. 56, pp. 2673—2676 (1978)] have reported the conversion of 2-vinylpyridine to 2-(1,2-epoxyethyl)pyridine N-oxide by the action of *m*-chloroperbenzoic acid on 2-vinylpyridine.

The present invention provides a process for the preparation of a compound of the formula:—

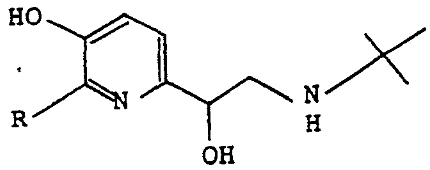

(I)

or a pharmaceutically acceptable acid addition salt thereof, wherein R is hydrogen, methyl or hydroxymethyl, which comprises treating a compound of the formula:—

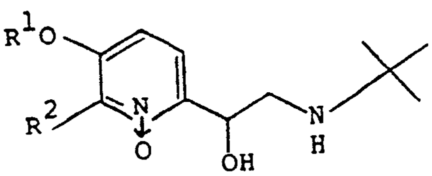

(V)

wherein $R^1$ is benzyl and $R^2$ is hydrogen or methyl, or $R^1$ and $R^2$ are taken together and are

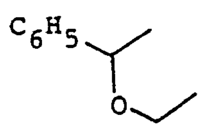

,

with hydrogen; or, alternatively, to produce a compound in which R is methyl or hydroxymethyl, solvolyzing the compound of the formula (V) in which $R^1$ and $R^2$ are taken together, followed by treatment with hydrogen; followed by, optionally, conversion of the product (I) into a pharmaceutically acceptable acid addition salt by reaction with a suitable acid.

In addition the invention includes the following process:—

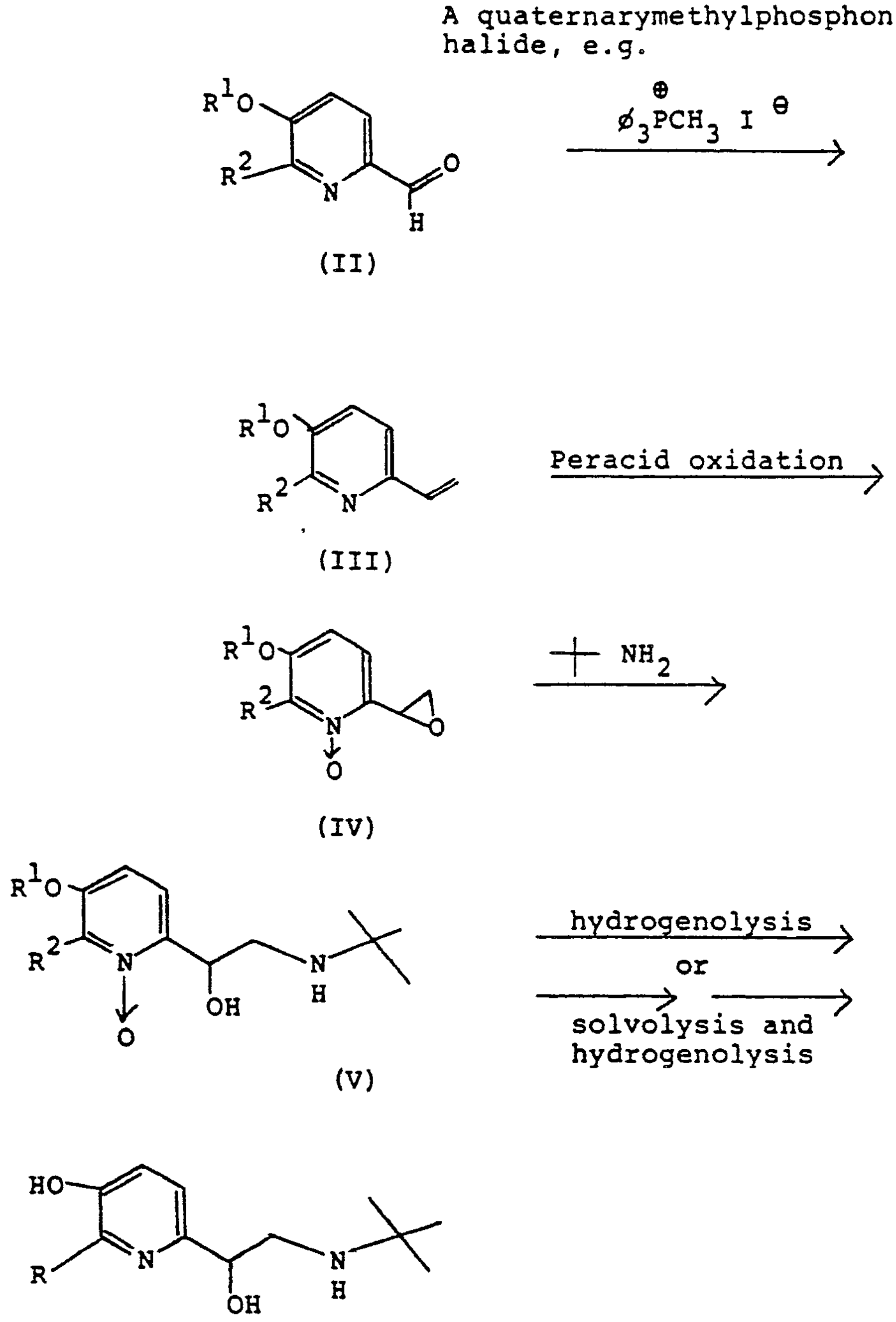


(II)

(III)

(IV)

(V)

(I)

where R, $R^1$, and $R^2$ are as defined hereinafter.

The invention also includes the novel intermediates of the formulae (III), (IV) and (V).

In the above reaction schemes, R is hydrogen, methyl or hydroxymethyl and $R^1$ is benzyl and $R^2$ is hydrogen or methyl; or $R^1$ and $R^2$ are taken together and are

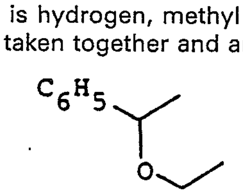 ,

This improved process avoids co-production of noxious sulfur compounds in the epoxidation step, permits epoxide opening under milder conditions, thus affording better quality intermediate and generally permits lower catalyst levels when hydrogenolysis is a later process step.

The process of the present invention has been summarized above in flowsheet form. The process is hereinafter described in greater detail, while referring to the flow diagram above.

The conversion of pyridinecarbaldehyde (II) to the vinylpyridine (III) is typically accomplished by the action of a molar excess (e.g. 2 equivalents) of a quaternarymethylphosphonium halide such as methyltriphenylphosphonium iodide in a reaction-inert organic solvent such as toluene in the presence of about one molar equivalent of sodium hydroxide at 0—50°C., conveniently at ambient temperature.

The conversion of the vinylpyridine (III) to the epoxide-N-oxide (IV) is typically accomplished by the action of at least two molar equivalents of peracid, conveniently *m*-chloroperbenzoic acid, in a reaction-inert solvent such as methylene chloride at a temperature of 10—60°C., conveniently at the reflux temperature of methylene chloride. The epoxide-N-oxide (IV) is alternatively prepared by peracid oxidation of epoxide compounds of the formula

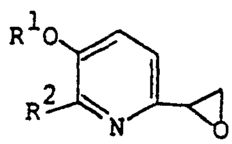

(IX)

wherein $R^1$ and $R^2$ are as hereinbefore defined. Preparation of the compound wherein $R^1$ is benzyl and $R^2$ is hydrogen is described in the Specific Examples detailed below. The compound wherein $R^1$ and $R^2$ are taken together is described in the above U.S. Patents to Nakanishi.

The conversion of the epoxide-N-oxide (IV) to the amine (V) is typically accomplished using an excess of *tert*-butylamine, optionally in the presence of an inert diluent such as methanol, at 20 to 70°C. In comparison to the corresponding *tert*-butylamine epoxide opening of a compound of the formula (IX), relatively mild conditions (lower temperature, shorter reaction time, less excess of reagent) are required, resulting in lowered cost and generally cleaner intermediate product.

In any case, the final stage of Process A, conversion of the amine (V) to the desired end-products (I), can be accomplished by hydrogenolysis over a noble metal catalyst in a reaction-inert solvent. The temperature of the hydrogenolysis is not critical, e.g. 0—100°C. can be used, but is conveniently carried out at ambient temperature avoiding the costs of cooling or heating. The pressure of the hydrogenation is also not critical (e.g. pressures of subatmospheric to 1378951 Pa or more can be used), but relatively low pressures (e.g. 137895—482633 Pa) are generally used, since the reaction proceeds at a reasonable rate without requiring the more elaborate equipment characteristic of high pressure hydrogenations. The noble metal catalysts employed in the present invention include platinum, palladium, ruthenium and rhodium, either of the supported or non-supported type, as well as the known catalytic compounds thereof such as oxides, chlorides. Examples of suitable catalyst supports include carbon and barium sulfate. In the present instance a preferred catalyst is palladium on carbon (1 to 10% of Pd by weight). The hydrogenation solvent is also not critical. It should not react with reactants or product (i.e. it should be reaction inert). Suitable solvents include ($C_1$—$C_4$) alcohols, particularly methanol which can serve as solvent for formation of the hydrochloride salt which is a a common form of pirbuterol. Other solvents such as tetrahydrofuran or dioxane can, of course, be used either alone or in combination with each other and/or the above mentioned alcohols. Higher boiling solvents, (e.g. ethylene glycol, diglyme) can also be used, but are not favored because more energy is required to removed them from the reaction mixture. In order to minimize or avoid hydrogenolysis of the 1-hydroxy-2-tert-butylaminoethyl side chain to a 2-tert-butylethyl group, it is preferred that the hydrogenolysis be carried out in the presence of a small amount of water (e.g. from 1 to 30 molar equivalents). In the particular case of preparing pirbuterol, the reaction is interrupted when the theoretical amount of hydrogen is consumed. If desired hydrogenation can be continued (optimally in the presence of acid, e.g. HCl) so as to carry out the following transformation:

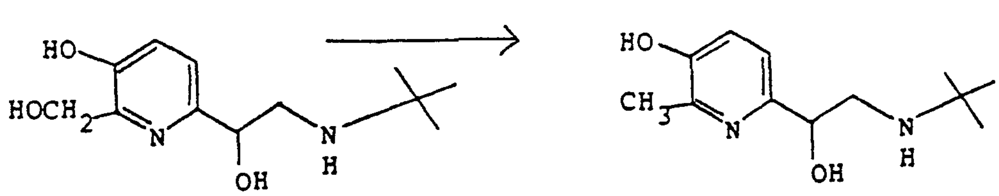

(I, R=$HOCH_2$) (I, R=$CH_3$)

When $R^1$ and $R^2$ are taken together, the conversion of the amine (V) is alternatively carried out by two stage solvolysis and hydrogenation as follows:

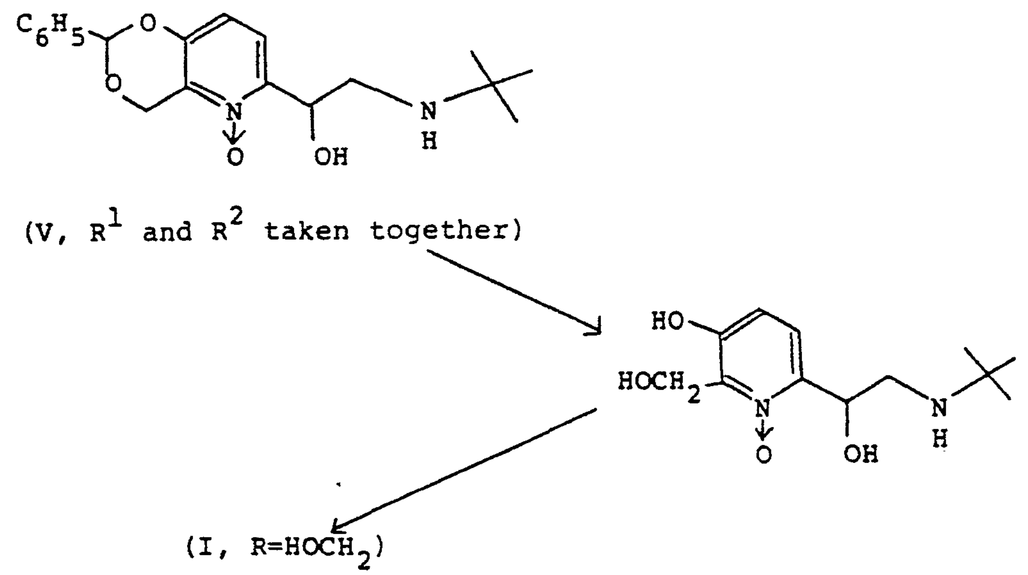

(V, $R^1$ and $R^2$ taken together)

(I, R=$HOCH_2$)

(XI)

The solvolysis is carried out in aqueous or alcoholic media, with or without cosolvents, usually in the presence of an acid catalyst. Particularly suitable is a combination of methanol and hydrogen chloride from which the intermediate is readily isolated in the form of the hydrochloride salt. Temperature is not critical; e.g. temperature of 0—50°C. or higher can be used, but the reaction is conveniently carried out at ambient temperature. Acid catalyzed solvolysis is preferred over base catalyzed solvolysis, since the products have some tendency to degrade under basic conditions. When solvolysis is used to remove the benzhydryl group, the final stage hydrogenolysis is carried out according to the methods detailed above, preferably on the free base form compound (XI) in order to avoid (unless desired) over hydrogenation of the 6-hydroxymethyl group to methyl.

The present invention is illustrated by the following Examples:

Example

A. 2-Phenyl-6-vinyl-4H-1,3-dioxino[5,4-b]pyridine (II, $R^1$ and $R^2$ taken together)

In a two liter 3-neck, round bottom flask, fitted with a mechanical stirrer, a thermometer and a reflux condenser, a heterogeneous mixture of 2-phenyl-4H-1,3-dioxino[5,4-b]pyridine-6-carbaldehyde (24.1 g., 0.1 mole) sodium hydroxide (24.0 g., 0.6 mole) and methyltriphenylphosphonium bromide (71.5 g., 0.2 mole) in toluene (200 ml.) and water (600 ml.) was stirred at room temperature for 24 hours. The toluene layer was separated and the aqueous layer was washed with toluene (100 ml.). The combined toluene layers were dried over anhydrous magnesium sulfate. The toluene solution was then passed through a 10 mm. pad of silica gel which was then washed with 1 liter of ether/hexane solution (v/v 3:2). Evaporation of the solvents on a rotary evaporation *in vacuo* gave the crystalline 2-phenyl-6-vinyl-4H-1,3-dioxino[5,4-b]pyridine, 14.3 g. (59%); m.p. 54—57°C.; mass spec (70 eV) *m/e* 211 ($M^+$), 134, 133, 132, 106, 105, 104.

| | | | |
|---|---|---|---|
| Anal. Calcd. for $C_{15}H_{13}NO_2$: | C, 75.29; | H, 5.48; | N, 5.86 |
| Found: | C, 75.42; | H, 5.01; | N, 6.01 |

B. 6-(1,2-Epoxyethyl)-2-phenyl-4H-1,3-dioxino[5,4-b]pyridine N-oxide (III, $R^1$ and $R^2$ taken together)

*Method A.*

In a 4-neck 500 ml., round bottom flask, fitted with a reflux condenser, a mechanical stirrer/magnetic stirring bar and a thermometer, was placed 2-phenyl-6-vinyl-4H-1,3-dioxino[5,4-b]pyridine (8.0 g., 0.033 mole) in methylene chloride (250 ml.). The solution was treated portion wise with *m*-chloroperbenzoic acid (20.0 g., 0.116 mole). When all of the peracid was added, the solution was heated at reflux overnight. Upon cooling *m*-chlorobenzoic acid crystallized from the reaction mixture and was filtered off. The filtrate was washed with aqueous sodium bicarbonate solution, dried over anhydrous magnesium sulfate and evaporated *in vacuo* to give the crude product (5.1 g.) which was recrystallized from ethyl acetate to give 6-(1,2-epoxyethyl)-2-phenyl-4H-1,3-dioxino[5,4-b]pyridine N-oxide (1.9 g., 21%), m.p. 180—181.5°C. (dec.); NMR ($CDCl_3$)delta 7.28 (m, 5H), 6.82 (q, 2H), 5.82 (s, 1H), 5.05 (s, 2H), 4.40 (d of d, 1H),

3.18 (d of d, 1H), 2.97 (d of d, 1H); mass spec (70 eV), *m/e* 271 ($M^+$), 241 (M—O, $CH_2$), 206, 165, 149, 132, 107, 105, 101, 82, 80, 79, 77.

| | | | |
|---|---|---|---|
| Anal. Calcd. for $C_{15}H_{13}NO_4$: | C, 66.41; | H, 4.83; | N, 5.16 |
| Found: | C, 66.36; | H, 4.43; | N, 4.79 |

*Method B.*

In a two liter 4-neck round bottom flask fitted with a thermometer, a magnetic stirring bar/stirrer and a reflux condenser was placed 6-(1,2-epoxyethyl)-2-phenyl-4H-1,3-dioxino[5,4-b]pyridine (125.7 g., 0.49 mole) in methylene chloride (1 liter). In several portions, *m*-chloroperbenzoic acid (109 g., 0.63 mole) was added to the stirred solution. Over a 30 minute period an exotherm to 32°C. was observed. The reaction mixture was allowed to stir at room temperature for 24 hours during which *m*-chlorobenzoic acid precipitated. After removing the acid by filtration, the filtrate was washed with aqueous sodium bicarbonate, dried over anhydrous magnesium sulfate, filtered and evaporated. The residual solid was recrystallized from isopropanol to give the N-oxide 65.5 g. (49%); m.p. 182—184°C. (dec); NMR identical with epoxy N-oxide prepared by Method A of this Example.

C. 6-(1-Hydroxy-2-*tert*-butylaminoethyl)-2-phenyl-4H-1,3-dioxino[5,4-b]pyridine N-oxide (IV, $R^1$ and $R^2$ are taken together)

To 15.0 g. (0.055 mole) of 6-(1,2-epoxyethyl)-2-phenyl-4H-1,3-dioxino[5,4-b]pyridine N-oxide in 200 ml. of methanol was added 30 ml. *tert*-butylamine and the resulting reaction mixture was heated to the reflux temperature for 3 hours. Solvent and excess *tert*-butylamine were removed *in vacuo* to give 13.4 g. (73.6%) of 6-(1-hydroxy-2-*tert*-butylaminoethyl)-2-phenyl-4H-1,3-dioxino[5,4-b]pyridine N-oxide after recrystallization from ethyl acetate: m.p. 165—168°C.

| | | | |
|---|---|---|---|
| Anal. Calcd. for $C_{19}H_{24}N_2O_4$: | C, 66.20; | H, 6.97; | N, 8.13 |
| Found: | C, 66.12; | H, 6.59; | N, 8.11 |

D. 3-Hydroxy-6-(1-hydroxy-2-*tert*-butylaminoethyl)-2-hydroxymethylpyridine N-oxide Dihydrochloride

Gaseous hydrogen chloride was bubbled into a solution of 5.0 g. (0.0145 mole) of 6-(1-hydroxy-2-*tert*-butylaminoethyl)-2-phenyl-4H-1,3-dioxino[5,4-b]pyridine N-oxide in 100 ml. of methanol at 10°C. After stirring overnight the solvent was removed *in vacuo*. The residue was triturated with ethyl acetate and filtered to give 3-hydroxy-6-(1-hydroxy-2-*tert*-butylaminoethyl)-2-hydroxymethylpyridine N-oxide dihydrochloride (3.9 g., 81%); m.p. 177—179°C. (dec.).

| | | | |
|---|---|---|---|
| Anal. Calcd. for $C_{12}H_{22}N_2O_4Cl_2$: | C, 43.77; | H, 6.73; | N, 8.51 |
| Found: | C, 43.76; | H, 6.53; | N, 8.49 |

E. 3-Hydroxy-6-(1-hydroxy-2-*tert*-butylaminoethyl)-2-methylpyridine dihydrochloride

Into a 500 ml. Parr bottle there was introduced 13.4 g. (0.04 mole) of 3-hydroxy-6-(1-hydroxy-2-*tert*-butylaminoethyl)-2-hydroxymethylpyridine N-oxide dihydrochloride and 6.5 g. of 5% palladium on carbon catalyst in 150 ml. of methanol. Under a hydrogen pressure of 296475 Pa the mixture was shaken at room temperature for two days. The catalyst was removed by filtration under a nitrogen atmosphere followed by removal of the solvent *in vacuo* to give an oil which was triturated with isopropanol to induce crystallization. Filtration of the resulting solid afforded 3-hydroxy-6-(1-hydroxy-2-*tert*-butylaminoethyl)-2-methylpyridine dihydrochloride, 10.0 g. (85%) m.p. 226—228.5°C. (dec.); NMR (DMSO $d_6$)delta 7.80 (q, 2H, pyridyl H), 5.40 (br m, 1H); 3.30 (br m, 2H), 2.60 (s, 3H, 2-$CH_3$) and 1.35 (s, 9H, t-Bn).

| | | | |
|---|---|---|---|
| Anal. Calcd. for $C_{12}H_{22}N_2O_2Cl_2.0.5H_2O$: | C, 47.06; | H, 7.57; | N, 9.11 |
| Found: | C, 47.20; | H, 7.13; | N, 9.21 |

## Example 2

Pirbuterol Hydrochloride [3-Hydroxy-6-(1-hydroxy-2-*tert*-butylaminoethyl)-2-hydroxymethylpyridine Dihydrochloride]

Into a 500 ml. Parr bottle there were introduced 970 mg. (0.0028 mole) of 6-(1-hydroxy-2-*tert*-butylaminoethyl)-2-phenyl-4H-1,3-dioxino[5,4-b]pyridine N-oxide (Example 1C) and 450 mg. 5% palladium on carbon catalyst in 25 ml. of methanol. Under a hydrogen pressure of 275750 Pa the mixture was shaken at room temperature for 17 hours. The catalyst was removed by filtration under a nitrogen atmosphere, the methanol solution was diluted with an equal volume of ethyl acetate and hydrogen chloride gas was introduced into the solution. Without heating, the resulting solution was concentrated *in vacuo* until the product began to precipitate. After stirring at room temperature for several hours, the white solid was collected by filtration and air-dried to give pirbuterol hydrochloride (450 mg., 51%); m.p. 180—184°C. (dec.)

which is identical by thin-layer chromatography (ethyl acetate/methanol/diethylamine [30:15:5 (v/v)], and nuclear magnetic resonance spectroscopy to the product in U.S. Patent No. 3,700,681.

Example 3

A. 5-Benzyloxy-2-vinylpyridine

In a two liter 3-neck round bottom flask fitted with a mechanical stirrer, thermometer and a water-cooled reflux condenser was placed 23.4 g. (0.11 mole) of 5-benzyloxypyridine-2-carbaldehyde, 78.5 g. (0.22 mole) of methyltriphenylphosphonium bromide and 26.5 g. (0.66 mole) of sodium hydroxide pellets in 250 ml. of toluene and 665 ml. of water. The reaction mixture was stirred at room temperature for 15 hours. The toluene layer was separated and saved. The aqueous layer was washed with toluene and the combined toluene layers are filtered through a 4" × 6" pad of silica gel to remove triphenylphosphate. The pad was successively washed with toluene, then ether/hexane (3:2) to remove the desired product. The organic solutions were combined, dried over anhydrous magnesium sulfate, filtered and evaporated *in vacuo* to give 5-benzyloxy-2-vinylpyridine as a yellow oil (20.5 g., 73.7%); NMR ($CDCl_3$) 8.15 (m, 1H, $H_6$), 7.16 (s, 5H, $C_6\underline{H}_5$—), 7.00 (m, 2H, $H_3$ and $H_4$), 6.67 (d of d, 1H, olefin H), 5.95 (d of d, 1H, olefin H), 5.15 (d of d, 1H, olefin H) and 4.90 (3, 2H, $CH_2$).

B. 5-Benzyloxy-2-(1,2-epoxyethyl)pyridine N-Oxide

In a 3-neck 100 ml. round bottom flask fitted with a thermometer, magnetic stirrer/stirring bar and reflux condenser is placed 1.00 g. (0.005 mole) of 5-benzyoxy-2-vinylpyridine in 30 ml. of methylene chloride. To this vigorously stirred solution *m*-chloroperoxybenzoic acid (2.00 g., 0.01 mole) was added in one portion. The resulting solution was heated to reflux for 12 hours. Workup in the same manner as Example 2 gave 5-benzyloxy-2-(1,2-epoxyethyl)pyridine N-oxide [0.50 g., (42%)]; m.p. 108—110°C. (dec.); NMR ($CDCl_3$) delta 8.00 (d, 1H, $H_6$), 7.33 (m, 5H, phenyl H), 7.30—7.10 (m, 2H, $H_3$ and $H_4$), 5.05 (s, 2H, $\underline{CH_2}$ of benzyl), 4.47 (m, 1H, epoxide CH), 3.23 (d of d, 1H, epoxide CH) and 2.67 (m, 1H, epoxide H), mass spectrum (70 eV) *m/e* 243 ($M^+$), 152 ($M^+$—$CH_2C_6H_5$), 91 ($C_7H_7^+$).

C. 5-Benzyloxy-2-(1-hydroxy-2-*tert*-butylaminoethyl)pyridine N-oxide

To 0.50 g. (0.0042 mole) of [5-benzyloxy-2-(1,2-epoxyethyl)pyridine N-oxide] in 20 ml. of methanol was added 0.5 ml. *tert*-butylamine and the resulting mixture heated to the reflux temperature for 4 hours. Solvent and excess *tert*-butylamine were removed *in vacuo* to give 280 mg. (43%) of the desired product after recrystallization from ether/ethyl acetate; m.p. 148—150° (dec.). Mass spec (70 eV) *m/e* 300 (M—16), *m/e* 243 [M—$H_2NC(CH_3)_3$]; NMR (DMSO $d_6$)delta 7.90 (d, 1H, $H_6$), 7.30 (m, 6H, benzyl $C_6H_5$ and $H_4$), 7.00 (m, 1H, $H_3$),

5.07 (m, 3H, $\phi\underline{CH_2}$ and $C\underline{H}CH_2$ (with OH on CH)); 4.33 (m, 2H, —CH(OH)—$\underline{CH_2}$N)

and 1.13 [s, 9H, $C(C\underline{H}_3)_3$]; ir (KBr) 1171 ($N^+$—$O^-$).

D. 5-Hydroxy-2-(1-hydroxy-2-*tert*-butylaminoethyl)pyridine

Into a 250 ml. Parr bottle there were introduced 250 mg. (0.79 mmole) of 5-benzyloxy-2-(1-hydroxy-2-*tert*-butylaminoethyl)pyridine N-oxide and 125 mg. of 5% palladium on carbon in 25 ml. of methanol. Under a hydrogen pressure of 310264 Pa, the mixture was shaken at room temperature for 6 hours. The catalyst was removed by filtration under a nitrogen atmosphere followed by removal of the solvent *in vacuo* to give the product which was triturated with isopropanol and filtered. In this manner, there was obtained 175 mg. (75%) of 5-hydroxy-2-(1-hydroxy-2-*tert*-butylamino-ethyl)pyridine as white crystals, m.p. 165—168°C. (decomp).

**Claims for the Contracting States: BE CH DE FR GB IT LI LU NL SE**

1. A process for the preparation of a compound of the formula:—

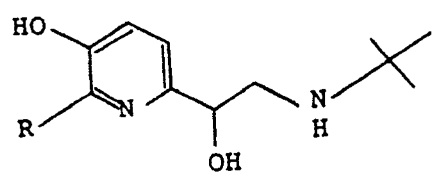

(I)

or a pharmaceutically acceptable acid addition salt thereof, wherein R is methyl or hydroxymethyl, which comprises the steps of

(a) oxidizing a vinylpyridine of the formula

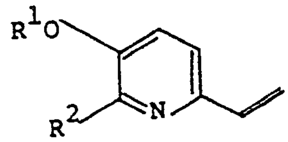

(III)

wherein $R^1$ is benzyl and $R^2$ is methyl, or $R^1$ and $R^2$ are taken together and are

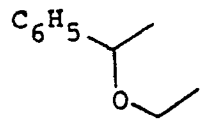

,

with a peracid and

(b) reacting the resulting epoxide-N-oxide of the formula

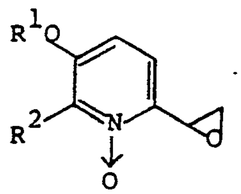

(IV)

wherein $R^1$ and $R^2$ are as defined above, with *tert*-butylamine, and

(c) treating the resulting compound of formula:—

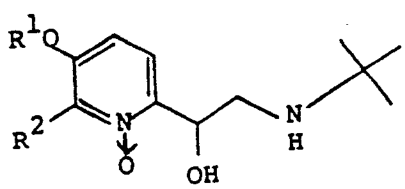

(V)

with hydrogen; or, alternatively, to produce a compound in which R is hydroxymethyl, solvolyzing the compound of the formula (V) in which $R^1$ and $R^2$ are taken together, followed by treatment with hydrogen; followed by, optionally, conversion of the product (I) into a pharmaceutically acceptable acid addition salt but reaction with a suitable acid.

2. A process according to claim 1 wherein the compound of the formula (III) is prepared by reacting a pyridinecarbaldehyde of the formula

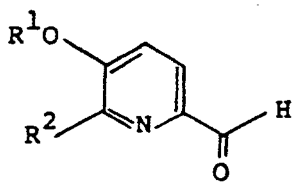

(II)

wherein $R^1$ and $R^2$ are as defined in claim 1, with a quaternary-methylphosphonium halide.

3. A process according to any one of the preceding claims, wherein the solvolysis is carried out using a combination of methanol and hydrogen chloride.

4. A process according to any one of the preceding claims, wherein the treatment with hydrogen is carried out over a noble metal catalyst, preferably palladium.

5. A process according to any one of the preceding claims, characterised in that $R^1$ and $R^2$ are taken together and are

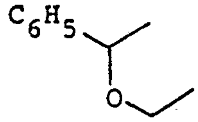

,

and wherein in the product (I) R is hydroxymethyl.

6. A compound of the formula

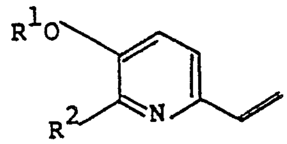

(III)

wherein $R^1$ is benzyl and $R^2$ is methyl; or $R^1$ and $R^2$ are taken together and are

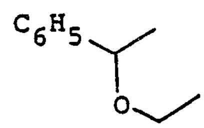

,

7. A compound of the formula

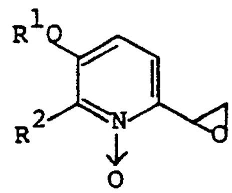

(IV)

wherein $R^1$ is benzyl and $R^2$ is methyl; or $R^1$ and $R^2$ are taken together and are

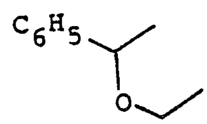

,

8. A compound of the formula

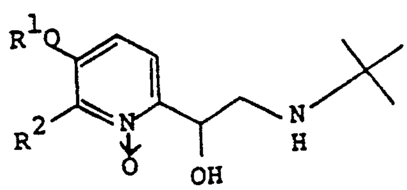

(V)

wherein $R^1$ is benzyl and $R^2$ is methyl; or $R^1$ and $R^2$ are taken together and are

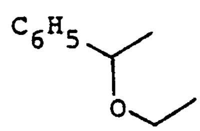

,

**Claims for the Contracting State: AT**

1. A process for the preparation of a compound of the formula:—

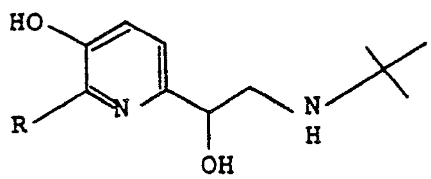

(I)

or a pharmaceutically acceptable acid addition salt thereof, wherein R is methyl or hydroxymethyl, which comprises the steps of

(a) oxidizing a vinylpyridine of the formula

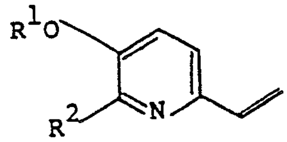

(III)

wherein $R^1$ is benzyl and $R^2$ is methyl, or $R^1$ and $R^2$ are taken together and are

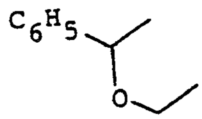

,

with a peracid and

(b) reacting the resulting epoxide-N-oxide of the formula

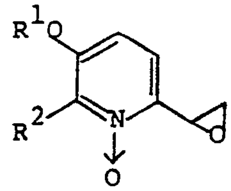

(IV)

wherein $R^1$ and $R^2$ are as defined above, with *tert*-butylamine, and

(c) treating the resulting compound of formula:—

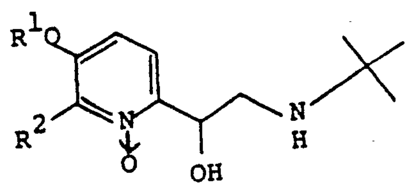

(V)

with hydrogen; or, alternatively, to produce a compound in which R is hydroxymethyl, solvolyzing the compound of the formula (V) in which $R^1$ and $R^2$ are taken together, followed by treatment with hydrogen; followed by, optionally, conversion of the product (I) into a pharmaceutically acceptable acid addition salt but reaction with a suitable acid.

2. A process according to claim 1 wherein the compound of the formula (III) is prepared by reacting a pyridinecarbaldehyde of the formula

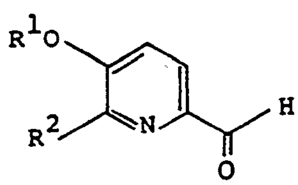

(II)

wherein $R^1$ and $R^2$ are as defined in claim 1, with a quaternary-methylphosphonium halide.

3. A process according to any one of the preceding claims, wherein the solvolysis is carried out using a combination of methanol and hydrogen chloride.

4. A process according to any one of the preceding claims, wherein the treatment with hydrogen is carried out over a noble metal catalyst, preferably palladium.

5. A process according to any one of the preceding claims, characterised in that $R^1$ and $R^2$ are taken together and are

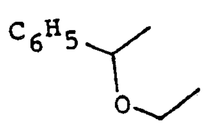

,

and wherein in the product (I) R is hydroxymethyl.

**Patentansprüche für die Vertragsstaaten: BE CH DE FR GB IT LI LU NL SE**

1. Verfahren zur Herstellung einer Verbindung der Formel

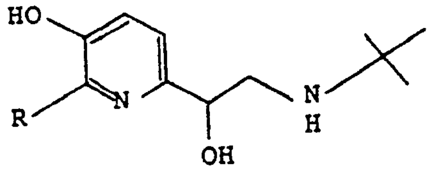

(I)

oder eines pharmazeutisch annehmbaren Säureadditionssalzes hievon, worin R Methyl oder Hydroxymethyl bedeutet, welches die Schritte des

(a) Oxidierens eines Vinylpyridins der Formel

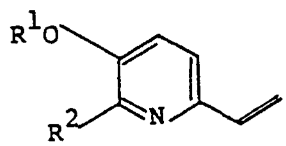

(III)

worin $R^1$ Benzyl ist und $R^2$ Methyl bedeutet oder $R^1$ und $R^2$ zusammen genommen sind und

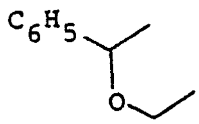

,

darstellen, mit einer Persäure,

(b) des Umsetzens des erhaltenen Epoxid-N-oxids der Formel

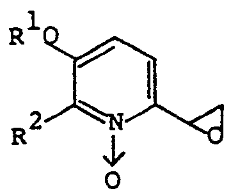

(IV)

worin $R^1$ und $R^2$ wie oben definiert sind, mit tert.Butylamin und

(c) des Behandelns der erhaltenen Verbindung der Formel

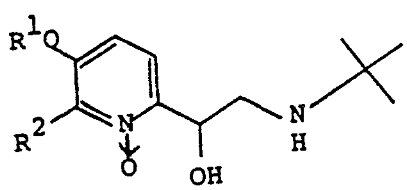

(V)

mit Wasserstoff oder aber zum Herstellen einer Verbindung, worin R Hydroxymethyl ist, des Solvolysierens der Verbindung der Formel (V), worin $R^1$ und $R^2$ miteinander genommen sind, gefolgt von der Behandlung mit Wasserstoff; gefolgt gegebenenfalls von der Überführung des Produktes (I) in ein pharmazeutisch annehmbares Säureadditionssalz durch Reaktion mit einer geeigneten Säure umfaßt.

2. Verfahren nach Anspruch 1, worin die Verbindung der Formel (III) durch Umsetzen eines Pyridincarbaldehyds der Formel

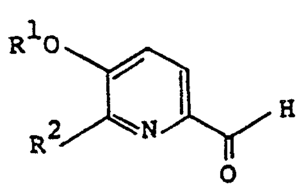

(II)

worin $R^1$ und $R^2$ wie in Anspruch 1 definiert sind, mit einem quaternären Methylphosphoniumhalogenid hergestellt wird.

3. Verfahren nach einem der vorhergehenden Ansprüche, worin die Solvolyse unter Verwendung einer Kombination von Methanol und Chlorwasserstoff durchgeführt wird.

4. Verfahren nach einem der vorhergehenden Ansprüche, worin die Behandlung mit Wasserstoff über einem Edelmetallkatalysator, vorzugsweise Palladium, durchgeführt wird.

5. Verfahren nach einem der vorhergehenden Ansprüche, dadurch gekennzeichnet, daß $R^1$ und $R^2$ miteinander genommen sind und

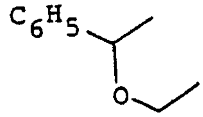

,

bedeuten und wobei im Produkt (I) R die Bedeutung Hydroxymethyl hat.

6. Eine Verbindung der Formel

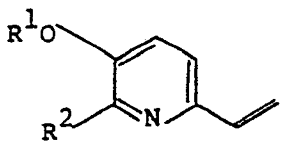

(III)

worin $R^1$ Benzyl ist und $R^2$ Methyl bedeutet oder $R^1$ und $R^2$ miteinander genommen sind und

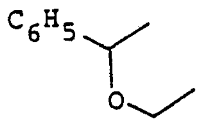

,

bedeuten.

7. Eine Verbindung der Formel

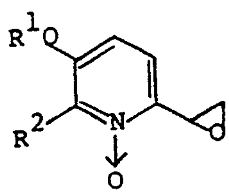

(IV)

worin $R^1$ Benzyl ist und $R^2$ Methyl darstellt oder $R^1$ und $R^2$ miteinander genommen sind und

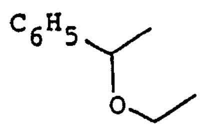

,

bedeuten.

8. Eine Verbindung der Formel

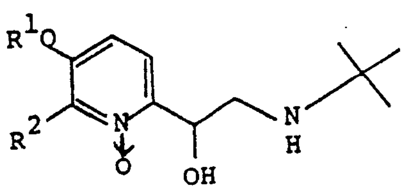

(V)

worin $R^1$ Benzyl ist und $R^2$ Methyl bedeutet oder $R^1$ und $R^2$ miteinander genommen sind und

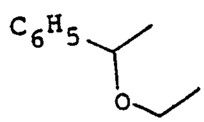

,

bedeuten.

**Patentansprüche für den Vertragsstaat: AT**

1. Verfahren zur Herstellung einer Verbindung der Formel

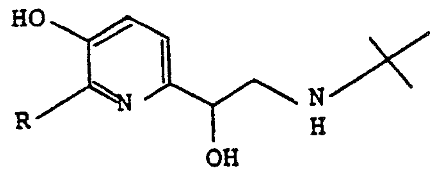

(I)

oder eines pharmazeutisch annehmbaren Säureadditionssalzes hievon, worin R Methyl oder Hydroxymethyl bedeutet, welches die Schritte des

(a) Oxidierens eines Vinylpyridins der Formel

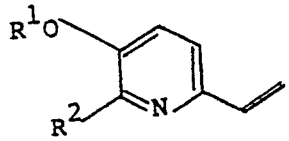

(III)

worin $R^1$ Benzyl ist und $R^2$ Methyl bedeutet oder $R^1$ und $R^2$ zusammen genommen sind und

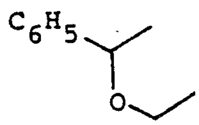

,

darstellen, mit einer Persäure,

(b) des Umsetzens des erhaltenen Epoxid-N-oxids der Formel

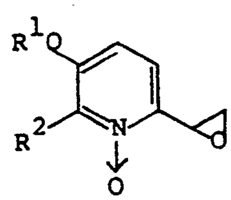

(IV)

worin $R^1$ und $R^2$ wie oben definiert sind, mit tert.Butylamin und

(c) des Behandelns der erhaltenen Verbindung der Formel

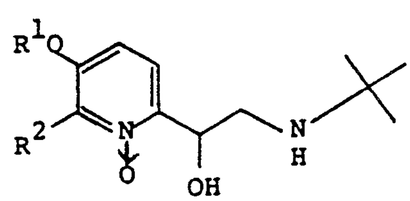

(V)

mit Wasserstoff oder aber zum Herstellen einer Verbindung, worin R Hydroxymethyl ist, des Solvolysierens der Verbindung der Formel (V), worin $R^1$ und $R^2$ miteinander genommen sind, gefolgt von der Behandlung mit Wasserstoff; gefolgt gegebenenfalls von der Überführung des Produktes (I) in ein pharmazeutisch annehmbares Säureadditionssalz durch Reaktion mit einer geeigneten Säure umfaßt.

2. Verfahren nach Anspruch 1, worin die Verbindung der Formel (III) durch Umsetzen eines Pyridincarbaldehyds der Formel

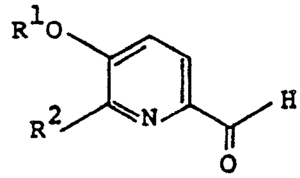

(II)

worin $R^1$ und $R^2$ wie in Anspruch 1 definiert sind, mit einem quaternären Methylphosphoniumhalogenid hergestellt wird.

3. Verfahren nach einem der vorhergehenden Ansprüche, worin die Solvolyse unter Verwendung einer Kombination von Methanol und Chlorwasserstoff durchgeführt wird.

4. Verfahren nach einem der vorhergehenden Ansprüche, worin die Behandlung mit Wasserstoff über einem Edelmetallkatalysator, vorzugsweise Palladium, durchgeführt wird.

5. Verfahren nach einem der vorhergehenden Ansprüche, dadurch gekennzeichnet, daß $R^1$ und $R^2$ miteinander genommen sind und

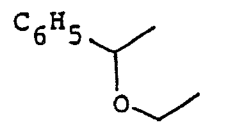

,

bedeuten und wobei im Produkt (I) R die Bedeutung Hydroxymethyl hat.

**Revendications pour les Etats contractants: BE CH DE FR GB IT LI LU NL SE**

1. Procédé de préparation d'un composé de formule:

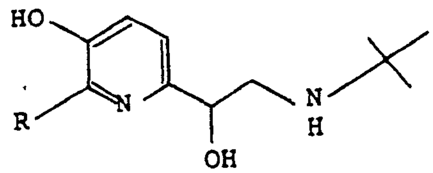

(I)

ou d'un sel d'addition d'acide pharmaceutiquement acceptable de ce composé, formule dans laquelle R est un groupe méthyle ou hydroxyméthyle, qui comprend les étapes consistant

(a) à oxyder une vinylpyridine de formule

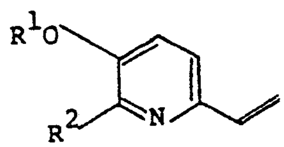

(III)

dans laquelle $R^1$ est un groupe benzyle et $R^2$ est un groupe méthyle, ou bien $R^1$ et $R^2$ forment conjointement un groupe

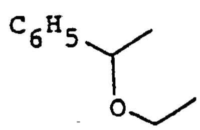

,

avec un peracide, et

(b) à faire réagir le N-oxyde d'époxyde résultant de formule

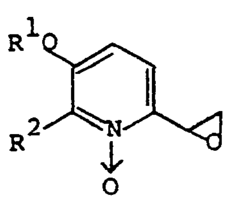

(IV)

dans laquelle $R^1$ et $R^2$ ont les définitions données ci-dessus, avec la tertio-butylamine, et

(c) à traiter le composé résultant de formule

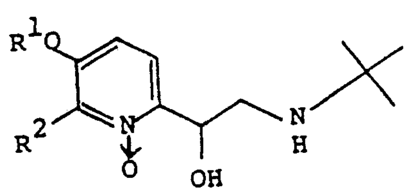

(V)

avec l'hydrogène; ou bien, à titre de variante, pour produire un composé dans lequel R est un groupe hydroxyméthyle, à solvolyser le composé de formule (V) dans laquelle $R^1$ et $R^2$ sont considérés ensemble, à

effectuer ensuite un traitement à l'hydrogène; après quoi, à titre facultatif, on transforme le produit (I) en un sel d'addition d'acide pharmaceutiquement acceptable par réaction avec un acide convenable.

2. Procédé suivant la revendication 1, dans lequel le composé de formule (III) est préparé par réaction d'un pyridinecarbaldéhyde de formule

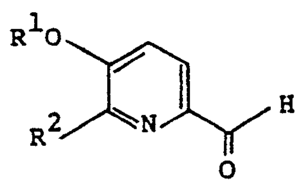

(II)

dans laquelle $R^1$ et $R^2$ ont la définition donnée dans la revendication 1, avec un halogénure de méthylphosphonium quaternaire.

3. Procédé suivant l'une quelconque des revendications précédentes, dans lequel la solvolyse est conduite en utilisant conjointement le méthanol et le chlorure d'hydrogène.

4. Procédé suivant l'une quelconque des revendications précédentes, dans lequel le traitement à l'hydrogène est effectué sur un catalyseur à base d'un métal noble, de préférence le palladium.

5. Procédé suivant l'une quelconque des revendications précédentes, caractérisé en ce que $R^1$ et $R^2$ sont considérés conjointement et représentent un groupe

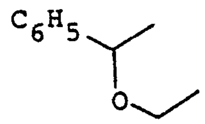

,

et dans le produit (I), R est un groupe hydroxyméthyle.

6. Composé de formule

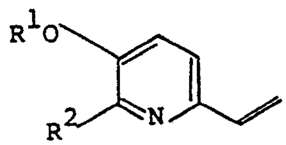

(III)

dans laquelle $R^1$ est un groupe benzyle et $R^2$ est un groupe méthyle; ou bien $R^1$ et $R^2$ sont considérés conjointement et forment un groupe

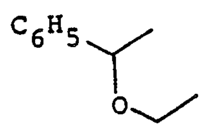

,

7. Composé de formule

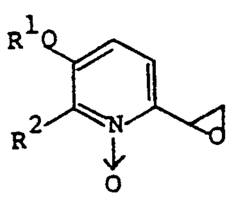

(IV)

dans laquelle $R^1$ est un groupe benzyle et $R^2$ est un groupe méthyle; ou bien $R^1$ et $R^2$ sont considérés conjointement et représentent un groupe

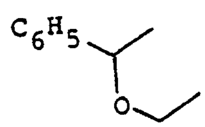

,

8. Composé de formule

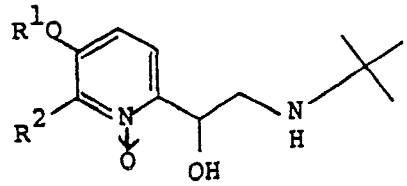

(V)

dans laquelle $R^1$ est un groupe benzyle et $R^2$ est un groupe méthyle; ou bien $R^1$ et $R^2$ sont considérés conjointement et forment un groupe

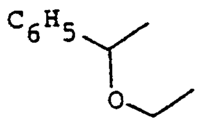

,

**Revendications pour l'Etat contractant: AT**

1. Procédé de préparation d'un composé de formule:

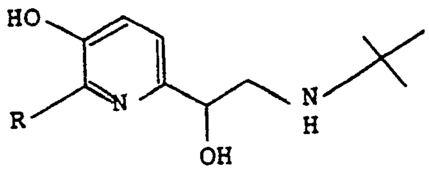

(I)

ou d'un sel d'addition d'acide pharmaceutiquement acceptable de ce composé, formule dans laquelle R est un groupe méthyle ou hydroxyméthyle, qui comprend les étapes consistant

(a) à oxyder une vinylpyridine de formule

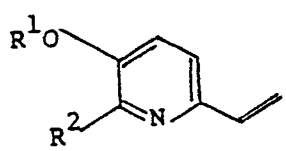

(III)

dans laquelle $R^1$ est un groupe benzyle et $R^2$ est un groupe méthyle, ou bien $R^1$ et $R^2$ forment conjointement un groupe

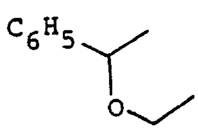

,

avec un peracide, et

(b) à faire réagir le N-oxyde d'époxyde résultant de formule

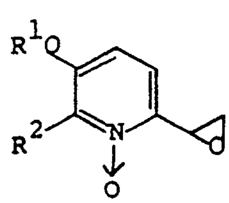

(IV)

dans laquelle $R^1$ et $R^2$ ont les définitions données ci-dessus, avec la tertio-butylamine, et

(c) à traiter le composé résultant de formule

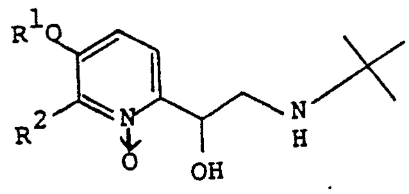

(V)

avec l'hydrogène; ou bien, à titre de variante, pour produire un composé dans lequel R est un groupe hydroxyméthyle, à solvolyser le composé de formule (V) dans laquelle $R^1$ et $R^2$ sont considérés ensemble, à effectuer ensuite un traitement à l'hydrogène; après quoi, à titre facultatif, on transforme le produit (I) en un sel d'addition d'acide pharmaceutiquement acceptable par réaction avec un acide convenable.

2. Procédé suivant la revendication 1, dans lequel le composé de formule (III) est préparé par réaction d'un pyridinecarbaldéhyde de formule

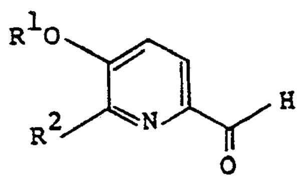

(II)

dans laquelle $R^1$ et $R^2$ ont la définition donnée dans la revendication 1, avec un halogénure de méthyl-phosphonium quaternaire.

3. Procédé suivant l'une quelconque des revendications précédentes, dans lequel la solvolyse est conduite en utilisant conjointement le méthanol et le chlorure d'hydrogène.

4. Procédé suivant l'une quelconque des revendications précédentes, dans lequel le traitement à l'hydrogène est effectué sur un catalyseur à base d'un métal noble, de préférence le palladium.

5. Procédé suivant l'une quelconque des revendications précédentes, caractérisé en ce que $R^1$ et $R^2$ sont considérés conjointement et représentent un groupe

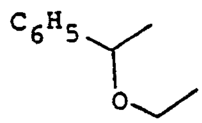

,

et dans le produit (I), R est un groupe hydroxyméthyle.